# EUROPEAN PATENT APPLICATION

(11) **EP 4 144 749 A1**
(43) Date of publication of application: **08.03.2023**
(21) Application number: 20933040.6
(22) Date of filing: 01.05.2020
(51) Int. Cl.: C07K 1/14

(54) **PURIFICATION METHOD FOR CHARGED SUBSTANCE**

(71) Applicant: Hoya Technosurgical Corporation, Tokyo 160-0004 (JP)
(72) Inventor: YOSHITAKE Tomohiko, Tokyo 160-0004 (JP)
(74) Representative: Diehl & Partner
(86) International application number: PCT/JP2020/018368
(87) International publication number: WO 2021/220500

(57) **Abstract**

An object of the present invention is to suppress the variation of the elution position of a compound having a charged portion by a preservation liquid, in the purification of the compound, without carrying out the substitution step of the preservation liquid attached to the adsorbent used for the purification and the keeping step. A method for purifying a compound having a charged portion, the method comprising the steps of: preparing a composition containing a compound having a charged portion; preparing a buffer solution comprising a buffering agent and an alcohol, the buffer containing a calcium phosphate compound at least partially, having a buffer capacity in a range of pH 6.0 to pH 8.0, and being soluble in a polar solvent and insoluble in a non-polar solvent; preserving an adsorbent in the buffer solution; adsorbing the compound on the adsorbent by bringing the composition into contact with the adsorbent preserved in the buffer solution; and separating the compound from the adsorbent by gradient elution.

## Description

### FIELD OF THE INVENTION

The present invention relates to a purification method of a charged material, more specifically, to a purification method using adsorbent comprising a calcium phosphate compound with the suppressed or prevented variation of the resolution.

### BACKGROUND OF THE INVENTION

A calcium phosphate compound such as hydroxyapatite has excellent biocompatibility, and thus has been widely used as adsorbent in a column for liquid chromatography (adsorption apparatus) by which a charged material such as a protein included in a sample liquid is adsorbed and desorbed (for example, see Patent Reference 1).

In such a column for liquid chromatography, the separation of a charged material in a sample liquid is conducted by adsorbing the charged material on the adsorbent composed of a calcium phosphate compound, and then desorbing it from the adsorbent. The separation of the charged material in the sample liquid can be carried out multiple times by repeating the adsorption and desorption.

The separation of the charged material using a calcium phosphate compound adsorbent may be carried out repeatedly without intervals of time, or with the intervals of a certain period of time (preservation period) such as seven days or more after the previous separation. During a certain period of time before the next separation of the charged material, the adsorbent may be kept in a preservation liquid such as a high-concentration sodium hydroxide solution (for example, about 1.0 M) or a phosphate buffer solution (for example, about 0.4 M).

The preservation of a calcium phosphate compound adsorbent in a preservation liquid for a certain period of time undesirably changes the separation position (elution time) of a negatively charged material such as an acidic protein bound to the calcium site of the adsorbent, whereas no change is observed in the separation position (elution time) of a positively charged material such as a basic protein bound to the phosphate site of the adsorbent.

Therefore, by using the adsorbent which has been preserved in the preservation liquid such as a sodium hydroxide solution or a phosphate buffer solution, and fractionating an eluate flowing out of the column for liquid chromatography in predetermined amounts, the fraction in which a negatively charged material is separated undesirably shifts before and after the preservation period. In order to recover the desired negatively charged material, this necessitates the removal of the difference of the separation position of the fraction to be collected before and after the preservation period, requiring time and labor.

As a technique to solve such a problem, there is disclosed a method for suppressing the variation in the elution position of a negatively charged material before and after the preservation period by, after the step of preserving the column in a preservation liquid for a certain period of time, a step of changing the preservation liquid to a phosphate buffer solution having a relatively low concentration and keeping the adsorbent in it for a while (for example, see Patent Reference 2). However, because this method needs the additional step of using the phosphate buffer solution after the preservation step, improvement is necessary from the viewpoint of enhancing efficiency in the separation operation. Further improvement is necessary to prevent the change in the elution position of a positively charged material.

### PATENT REFERENCE

Patent Reference 1: JP 2013-534252 A
Patent Reference 2: WO 2019/224660

### OBJECT OF THE INVENTION

An object of the present invention is to suppress the variation of the elution position of a compound having a charged portion by a preservation liquid, in the purification of the compound, without carrying out the substitution step of the preservation liquid attached to the adsorbent used for the purification and the keeping step.

### SUMMARY OF THE INVENTION

As a preferred embodiment, the present invention includes any one of the following embodiments.
[A1] A method for purifying a compound having a charged portion, the method comprising the steps of:
   preparing a composition containing a compound having a charged portion;
   preparing a buffer solution comprising a buffering agent and an alcohol, the buffer containing a calcium phosphate compound at least partially, having a buffer capacity in a range of pH 6.0 to pH 8.0, and being soluble in a polar solvent and insoluble in a non-polar solvent;
   preserving the adsorbent in the buffer solution;
   adsorbing the compound on the adsorbent by bringing the composition into contact with the adsorbent preserved in the buffer solution; and
   separating the compound from the adsorbent by gradient elution.
[A2] The purification method described in A1, in which the adsorption step is carried out after an equilibration step using the same kind of a buffering agent as that of the buffering agent used in the adsorption step, without carrying out a substitution step of the buffer solution after the preservation step.
[A3] The purification method described in A1 or A2, wherein the charged material has a negatively charged portion.
[A4] The purification method described in any one of A1 to A3, wherein the charged material has a positively charged portion.
[A5] The purification method described in any one of A1 to A4, wherein the buffering agent is selected from the group consisting of MES, ADA, PIPES, ACES, cholamine chloride, BES, TES, HEPES, acetamido glycine, tricine, glycinamide, and bicine.
[A6] The purification method described in any one of A1 to A5, comprising a step of purifying the compound having the charged portion.

### EFFECTS OF THE INVENTION

An object of the present invention is to suppress variation of the elution position of a compound having a charged portion by a preservation liquid for preserving adsorbent, in the purification of the compound, without carrying out the substitution step of the preservation liquid and the keeping step.

### BRIEF DESCRIPTION OF THE DRAWINGS

Fig. 1 is a vertical cross-sectional view showing an example of an adsorption apparatus having adsorbent which can be applied to the purification method of the embodiment of the present invention.
Fig. 2 is a graph showing the result of elution in Example 1.
Fig. 3 is a graph showing the result of elution in Comparative Example 1.
Fig. 4 is a graph showing the result of elution in Comparative Example 2.
Fig. 5 is a graph showing the result of elution in Comparative Example 3.

### DESCRIPTION OF THE PREFERRED EMBODIMENTS

The purification method of the present invention will be described in detail below on the basis of the preferred embodiment shown in the attached drawing. First, before describing the purification method of the present invention, an example of an adsorption apparatus including adsorbent which can be applied to the purification method of the present invention will be described.

In the example described below, a charged material to be separated using the adsorption apparatus is a protein, that is, a negatively charged material is an acidic protein and a positively charged material is a basic protein.

### <Adsorption apparatus>

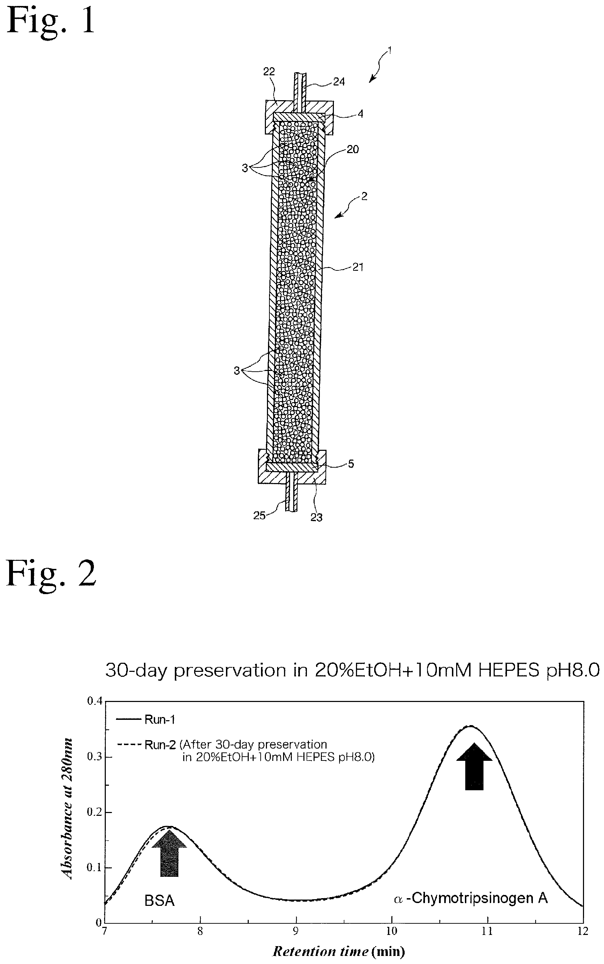

Fig. 1 is a vertical cross-sectional view showing an example of the adsorption apparatus including adsorbent which can be applied to the purification method of the present invention. It is noted that the upper side in Fig. 1 is referred to as an "inlet side," and the lower side in Fig. 1 is referred to as an "outlet side," hereafter.

Herein, the inlet side means a side through which a liquid such as a sample liquid (liquid including a sample), an eluent (phosphate buffer solution), etc. is supplied into the adsorption apparatus in the separation (purification) of the target protein. On the other hand, the outlet side means a side opposite to the inlet side, through which the liquid flows out from the adsorption apparatus.

The adsorption apparatus 1 shown in Fig. 1 for separating (purifying) the protein from the sample liquid has a column 2, particulate adsorbents (filler) 3, and two filter members 4, 5.

The column 2 is constituted by a column body 21, and caps (lid bodies) 22, 23 attached to the inlet-side end and the outlet-side end of the column body 21 respectively.

The Column body 21 is configured by, for example, a cylindrical member. Each component (member) of the column 2, which includes the column body 21, may be formed by, for example, various glass materials, various resin materials, various metal materials, various ceramic materials, etc., or arbitrary combinations thereof.

After the filter members 4, 5 are covered on the inlet-side opening and the outlet-side opening of the column body 21 respectively, the caps 22, 23 are screwed in the inlet-side end and the outlet-side end thereof respectively.

In the column 2 of the above configuration, an adsorbent filling space 20 is defined by the column body 21 and the filter members 4, 5. The adsorbent filling space 20 is filled at least partially (fully in this embodiment) with the adsorbents 3.

The capacity of the adsorbent filling space 20 is appropriately set according to the volume of the sample liquid, without being particularly limited, but it is preferably about 0.1 mL or more and about 100 mL or less, and more preferably about 1 mL or more and about 50 mL or less, per 1 mL of the sample liquid.

The Column body 21 equipped with the caps 22, 23 secures liquid tightness therebetween.

An inlet pipe 24 and an outlet pipe 25 are liquid-tightly fastened (fixed) to almost the centers of the caps 22, 23, respectively. The liquid is supplied to the adsorbents 3 through the inlet pipe 24 and the filter member 4. The liquid supplied to the adsorbents 3 passes through spaces (gaps) between the adsorbents 3, and then flows out of the column 2 through the filter member 5 and the outlet pipe 25. At this time, the protein to be separated (hereinafter, may be simply referred to as "protein"), and the other material which may include foreign protein other than the protein to be separated (hereinafter, may be simply referred to as "foreign protein"), in the sample liquid (sample), are separated on the basis of the difference of adsorptivity to the adsorbents 3 and the difference of affinity to the phosphate buffer solution.

Each filter member 4, 5 has a function of preventing the adsorbents 3 from flowing out of the adsorbent filling space 20. Each filter member 4, 5 is preferably formed by, for example, a stainless steel net filter or a sintered stainless steel filter.

The adsorbents 3 include a calcium phosphate compound at least partially. More preferably at least the surface of the adsorbent 3 is formed by a calcium phosphate compound. The protein to be separated is specifically adsorbed on the adsorbents 3 with its intrinsic adsorption (retention) power, and then separated and purified from the other material (including the foreign protein) according to the difference of their adsorption powers.

The calcium phosphate compound may be, for example, hydroxyapatite [Ca₁₀(PO₄)₆(OH)₂], TCP [Ca₃(PO4)₂], Ca₂P₂O₇, Ca(PO₃)₂, DCPD (CaHPO₄ • 2H₂O), Ca₄O(PO₄)₂, and their derivatives which are at least partially substituted with other atoms or atomic groups, or may be a combination of two or more thereof.

In general, proteins are classified into negatively charged acidic proteins containing a relatively large amount of acidic amino acids as their constituent amino acids, and positively charged basic proteins containing a relatively large amount of basic amino acids as their constituent amino acids.

The calcium phosphate compound has a positively charged Ca site and a negatively charged phosphate site in its crystal structure.

Accordingly, the acidic protein is adsorbed by the calcium phosphate compound with an ionic bond between the Ca site of the calcium phosphate compound and the acidic amino acid residue of the acidic protein. The basic protein is adsorbed by the calcium phosphate compound with an ionic bond between the phosphate site of the calcium phosphate compound and the basic amino acid residue of the basic protein. The binding (adsorbing) strength of the protein with the calcium phosphate compound differs according to the amount of charge of each protein (acidic protein and basic protein).

Therefore, when the adsorbents 3 composed of the calcium phosphate compound at least on the surfaces are used, it is possible to separate the protein (acidic protein and basic protein) and the other material (for example, foreign protein, etc.) using the difference of their adsorbing powers to the adsorbents 3.

Among the above, the calcium phosphate compound is preferably mainly composed of hydroxyapatite. Because hydroxyapatite has particularly similar components to those constituting a living body, it can prevent the deterioration (denaturation) of the protein in the adsorption and separation thereof. Further, there is an advantage that the protein can be specifically removed from the adsorbents 3, for example, by providing the phosphate buffer solution (eluent) with gradient of the salt concentration.

That the calcium phosphate compound is "mainly composed of" hydroxyapatite means that the amount of hydroxyapatite is about 50% by mass or more per 100% by mass of the total amount of the calcium phosphate compound. The amount of hydroxyapatite is more preferably about 60% by mass or more, further preferably about 70% by mass or more, further more preferably about 80% by mass or more, and most preferably about 90% by mass or more.

At least one of the hydroxyl groups of hydroxyapatite may be substituted with a fluorine atom. The hydroxyapatite substituted with the fluorine atom (hereinafter referred to as "fluoroapatite") can more reliably prevent a calcium atom (calcium ion) from being detached therefrom due to the presence of the fluorine atom (fluorine ion) in the crystal structure.

It is noted that the hydroxyapatite and the fluoroapatite with the substituted fluorine atom may be collectively called as "apatite," hereinafter.

As shown in Fig. 1, the form (shape) of the adsorbent 3 is preferably a particulate (granular) shape, and also may be, for example, a pellet shape (small block shape), a block shape (for example, a porous body shape whose adjacent voids communicate with each other, and a honeycomb shape), etc. Because the adsorbents 3 with a particulate shape have a large surface area, separation characteristics of the protein can be improved.

The average size of the particulate adsorbents 3 is, although not particularly limited, preferably about 0.5 µm to about 150 µm, and more preferably about 10 µm to about 80 µm. The adsorbents 3 with the above average size can reliably prevent the filter member 5 from clogging, as well as sufficiently secure their surface area.

The adsorbent 3 may be entirely composed of a calcium phosphate compound, or may be formed by a carrier (substrate) coated with a calcium phosphate compound on the surface. Among them, the adsorbent 3 entirely composed of a calcium phosphate compound is preferable, thereby further enhancing the adsorption power of the adsorbent 3. As a result, the column 2 suitable for separating a large quantity of the protein can be obtained.

The adsorbents 3 composed of a calcium phosphate compound entirely can be obtained, for example, by forming calcium phosphate compound particles (primary particles) using a wet synthesis method or a dry synthesis method, drying and granulating a slurry containing the calcium phosphate compound particles to obtain dried particles, and then sintering the dried particles.

On the other hand, the adsorbent 3 composed of a carrier coated with a calcium phosphate compound on the surface can be obtained, for example, by colliding (hybridizing) the dried particles with the carrier made of a resin, etc.

When the adsorbent filling space 20 is fully filled with the adsorbents 3 as in this embodiment, the adsorbents 3 preferably have almost the same composition at respective portions of the adsorbent filling space 20, thereby providing the adsorption apparatus 1 with a particularly excellent ability of separating (purifying) the protein.

It is noted that a portion of the adsorbent filling space 20 (for example, a portion on the side of the inlet pipe 24) may be filled with the adsorbents 3, with the other portion filled with other adsorbents.

### <Purification method>

The purification method of the protein using the adsorption apparatus 1 will be described below.

### [1A] Preparation step

A sample liquid (composition) containing the protein to be separated (purified) and the other material (contaminant) is prepared.

When the protein is genetically modified protein (monoclonal antibody, etc.), the sample liquid may include a secretion from mammals such as sheep, rabbits, chickens, etc., animals such as insects such as silkworms, animal cells such as CHO cells derived from Chinese hamster ovarian cells, microorganisms such as Escherichia coli, etc., into which a nucleic acid including a gene coding the protein is transferred, a cytosol component thereof, etc., and their arbitrary combination.

When the protein is natural protein, the sample liquid may include, for example, a blood (plasma) derived from various animals, a body fluid such as a lymph, a saliva and a nasal discharge, etc.

Among the protein, the acidic protein may be, for example, BSA, HAS, fibrinogen, pepsinogen, α-globulin, β-globulin, γ-globulin, etc. On the other hand, the basic protein may be, for example, lysozyme, thaumatin, cytochrome C, ribonuclease, trypsinogen, chymotrypsinogen, α-chymotrypsin, histone, protamine, polylysine, monellin, etc.

It is noted that the protein (acidic protein and basic protein) may be a variant obtained by replacing some amino acids of the amino acid sequence thereof with other amino acids.

As the sample liquid (composition containing a compound having a charged portion to be separated), the secretion from the microorganisms, etc. and the body fluid derived from animals may be used as they are or after delusion by a neutral buffer solution such as water and physiological saline, or filtration by a membrane such as a filter. Further, the sample liquid may contain plural kinds of the protein.

### [2A] Supply step

The obtained sample liquid is supplied to the adsorbents 3 through the inlet pipe 24 and the filter member 4, and passes through the inside of the column 2 (adsorption apparatus 1) so as to be brought into contact with the adsorbents 3.

As a result, the protein to be separated (purified) having a high adsorption ability on the adsorbents 3, and some (protein) of the contaminant (foreign protein etc.) which has a relatively high adsorption ability on the adsorbents 3, are retained in the column 2. The other of the contaminant which has a low adsorption ability on the adsorbents 3 flows out from the inside of the column 2 through the filter member 5 and the outlet pipe 25.

### [3A] Fractionation step

An eluent for eluting the protein such as a phosphate buffer solution is supplied into the column 2 through the inlet pipe 24. Then, an eluate flowing out from the inside of the column 2 through the outlet pipe 25 is fractionated (collected) in predetermined amounts. That is, the protein and the contaminant adsorbed on the adsorbents 3 respectively flow out in a dissolved state at different times according to the difference of their respective adsorption powers to the adsorbents 3, and then are collected (separated / purified) into the respective fractions.

Thus, the protein and the contaminant are specifically adsorbed on the adsorbents 3 with their respective intrinsic adsorption (retention) powers. That is, there is a difference between their adsorption powers to the adsorbents 3. According to the difference of the adsorption powers, the protein and the contaminant are separated and purified. By selectively collecting the fraction in which the protein to be separated (purified) is dissolved, the purified protein can be obtained.

The buffering agent used for the eluent is preferably a Good's buffer such as HEPES and MES, sulfate, and phosphate. Among them, a buffering agent having a sulfonic acid group and phosphate are more preferable. The buffering agent having a sulfonic acid group is preferably MOPS, MES, or HEPES. The phosphate may be, for example, sodium phosphate, potassium phosphate, lithium phosphate, ammonium phosphate, etc. Among them, sodium phosphate is more preferable. The eluent may further contain salt such as sodium chloride.

Although the higher pH of the phosphate buffer solution (solution in which phosphate is dissolved) is advantageous from the viewpoint of enhancing the elution capacity, the pH of the phosphate buffer solution is preferably almost the same as that of the buffering agent used for the adsorption (equilibration) in general. In addition, with the pH almost neutral, the protein is hardly denatured. Therefore, the pH value is set to, for example, preferably about 5.5 to 8.5, and more preferably about 6.5 to 7.5.

The temperature of the buffer solution (eluent) is preferably about 10 to 50°C, and more preferably about 20 to 35°C from the viewpoints of preventing degeneration (denaturation) of the protein to be separated and preventing precipitation of the phosphate buffering agent. Accordingly, with the buffer solution having the above pH range and temperature range, the recovery rate of the target protein can be improved. When the target protein to be separated is easily denatured, however, the temperature of the buffer solution may be lower than the above temperature range (around a refrigerating room temperature).

The salt concentration of the buffer solution is preferably 500 mM or less, and more preferably 400 mM or less. By carrying out the separation of the protein using the buffer solution with the above salt concentration, it is possible to prevent metal ions in the phosphate buffer solution from adversely affecting the protein.

The salt concentration of the buffer solution is more preferably about 1 to 400 mM. In addition, the salt concentration of the buffer solution is preferably changed continuously or stepwise in the separation of the protein, thereby improving efficiency in the separation of the protein.

The flow rate of the buffer solution is preferably about 0.1 to 10 mL/min, and more preferably about 1 to 5 mL/min. By separating the protein in the above flow rates, the target protein can be reliably separated, that is, high-purity protein can be obtained, without a long-time separation operation.

According the above operations, the protein is recovered in the predetermined fraction. Therefore, the protein to be separated is obtained in the predetermined fraction, and then purified.

Then, the column used in the steps 1A to 3A can be applied to the preservation step of the embodiment of the present invention as described below. Therefore, the steps 1A to 3A may be comprehended as a step for preparing a column to be applied to the purification method of the present invention. In the embodiment of the present invention, the column to be applied to the purification method is preferably the one which has been already used and thus contains or may contain materials by which the adsorption ability of adsorbents changed, or the one which contains adsorbents whose adsorption ability has been changed, even though unused, due to some reasons such as the presence of impurities.

### <Preservation>

### [4A] Preservation step

In the conventional method using the adsorption apparatus 1, the separation (purification) of protein is repeated by washing the adsorption apparatus 1 by eluting the other material (foreign protein, etc.) adsorbed on the adsorbents 3 using a high-concentration buffer solution (phosphate buffer solution, etc.) without an interval after the separation (purification) of the protein, and then using the adsorption apparatus 1 for the next separation of the protein again.

Alternatively, the adsorbents 3 which have been preserved for a certain period of time (preservation period) such as 1 to 30 days may be used for the separation of the protein in the adsorption apparatus 1. In the conventional method, during a certain period of time before the next separation of the charged material, the adsorbents 3 are usually kept in a preservation liquid such as a high-concentration sodium hydroxide solution (for example, about 1.0 M), a phosphate buffer solution (for example, about 0.4 M), etc., for the purpose of further elution of the other material, etc.

However, when the separation of the protein using the adsorption apparatus 1 is carried out after the preservation in a sodium hydroxide solution or a phosphate buffer solution for a certain period of time, the separation position (elution time) of the basic protein bound to the phosphate site does not vary, but the separation position (elution time) of the acidic protein bound to the calcium site sometimes varies from before the preservation.

On the other hand, when the preservation of the adsorbents 3 in a phosphate buffer solution (for example, a phosphate buffer solution of about 15 mM) is carried out for a much longer period of time than 7 days (for example, about 30 days), the elution position of the acidic protein (negatively charged material) does not vary, but the elution position of the basic protein (positively and negatively charged material) sometimes varies. Therefore, when the positively and negatively charged materials are purified by the method described in Patent Reference 2, it may be necessary to restore the elution position of the fractions from which the positively and negatively charged materials are to be recovered. It is noted that this problem has been found by the present inventors.

On the contrary, in the purification method of the present invention, by using, as a preservation liquid, a buffer solution including a buffering agent which contains a calcium phosphate compound at least partially, has a buffer capacity in a range of pH 6.0 to pH 8.0, and is soluble in a polar solvent and insoluble in a non-polar solvent, and an alcohol (step "4A"), it is possible to prevent the elution time from shifting without carrying out any additional step. The buffering agent may have a maximum buffer capacity in a range of pH 6.0 to pH 8.0.

The buffering agent contained in the buffer solution used as the preservation liquid is preferably selected from the group consisting of MES, ADA, PIPES, ACES, cholamine chloride, BES, TES, HEPES, acetamido glycine, tricine, glycinamide and bicine. These are so-called Good's buffers which have a buffer capacity in a range of pH 6.0 to pH 8.0, and are soluble in a polar solvent and insoluble in a non-polar solvent. Among them, the buffering agent is more preferably MES or HEPES, and most preferably HEPES.

The concentration of the buffering agent in the preservation liquid is preferably about 1.0 mM or more and about 100 mM or less, more preferably about 2.0 mM or more and about 50 mM or less, further preferably about 3.0 mM or more and about 30 mM or less, and further preferably about 4.0 mM or more and about 20 mM or less.

The preservation liquid includes an alcohol. The content of the alcohol is preferably 5 to 40% by volume, more preferably 10 to 30% by volume, and most preferably 15 to 25% by volume, per 100% by volume of the total amount of the preservation liquid. When the content of the alcohol is set to the above range, it is possible to prevent deterioration of the adsorbents, as well as to further suppress occurrence of bacteria, etc.

The alcohol is preferably a lower alcohol. The lower alcohol means a monovalent alcohol having 1 to 6 carbon atoms. The lower alcohol is not particularly limited as long as physiologically or pharmaceutically acceptable. The specific examples of the lower alcohol are, for example, ethanol, propanol, isopropanol, n-butyl alcohol, s-butyl alcohol, t-butyl alcohol, isobutyl alcohol, pentyl alcohol and hexyl alcohol. From the viewpoint of more remarkably exhibiting the effect of the present invention, the lower alcohol is preferably ethanol, propanol, isopropanol, n-butyl alcohol, s-butyl alcohol, t-butyl alcohol or isobutyl alcohol, more preferably ethanol or isopropanol, further preferably ethanol. The lower alcohol may be composed of one kind only or the combination of two or more kinds thereof.

In the purification method of the present invention, after preserving the adsorbents 3 for a certain period of time as described above, the variation of the adsorption ability (separation ability) of the protein to be adsorbed on the adsorbents 3 is suppressed. As a result, the variation of the elution time is suppressed without substituting the preservation liquid with the other buffering agent.

Although protein is used as an example of the charged material in the above description, the negatively charged material may be an acidic amino acid, DNA, RNA, a negatively charged liposome, etc., other than acidic protein. The positively charged material may be a basic amino acid, a positively charged cholesterol, a positively charged liposome, etc., other than basic protein.

Although the purification method of the present invention has been described above, the present invention is not restricted thereto.

The purification method of the present invention may be added with one or more steps without deviating from the technical idea of the present invention.

### EXAMPLE

The specific examples of the present invention will be described below.

### 1. Manufacture of adsorption apparatus

The adsorption apparatus was manufactured by filling the filling space of the stainless steel column (inner diameter 4.0 mm × length 100 mm) almost fully with hydroxyapatite (CHT 40 µm Type I available from Bio-Rad Laboratories, Inc.) as the adsorbents (filler).

### 2. Separation of protein (acidic protein and basic protein) by adsorption apparatus

### (Example 1)

### 2-1. Separation of protein by adsorption apparatus before preservation in preservation liquid

<1A> Bovine serum albumin (BSA) as an acidic protein and α-chymotrypsinogen A as a basic protein were dissolved into 1.0 mM sodium phosphate buffer solution (pH 6.8) to obtain a mixed liquid so that the content of BSA was 10 mg/mL and the content of α-chymotrypsinogen A was 5 mg/mL. Then, the mixed liquid was filtered through a 0.22 µm filter to obtain a sample liquid.
<2A> The adsorption apparatus was mounted on a chromatograph. The adsorption apparatus was washed with 1M NaOH, and then substituted with 0.4 M sodium phosphate buffer solution (pH 6.5, temperature 25°C). Then, 10 mM sodium phosphate buffer solution (pH 6.5, temperature 25°C) was supplied through the adsorption apparatus at a flow rate of 1.0 mL/min to equilibrate the adsorption apparatus.
<3A> 50 µL of the sample liquid was supplied into the adsorption apparatus at a flow rate of 1.0 mL/min, and then a concentration gradient was applied to the phosphate buffer solution (pH 6.5) so that the concentration increased from 10 mM to 400 mM (75%) for 15 minutes. The absorbance at 280 nm of the eluate (liquid flowing out from the inside of the adsorption apparatus) was measured. After the measurement, the adsorption apparatus was washed with 0.4 M sodium phosphate buffer solution (pH 6.5, temperature 25°C), and then washed with water.
<4A> 10 mM HEPES buffer solution (pH 8.0) containing 20% ethanol was supplied through the adsorption apparatus at a flow rate of 1.0 mL/min to immerse the adsorbents in the HEPES buffer solution. Then, the adsorbents were preserved in this state for 30 days.

### 2-2. Separation of protein by adsorption apparatus after preservation in preservation liquid

<1B> The adsorption apparatus was mounted on a chromatograph. Then, 10 mM sodium phosphate buffer solution (pH 6.5, temperature 25°C) was supplied through the adsorption apparatus at a flow rate of 1.0 mL/min to equilibrate the adsorption apparatus.
<2B> 50 µL of the same sample liquid as that prepared in the step <1A> was supplied into the adsorption apparatus at a flow rate of 1 mL/min, and then a concentration gradient was applied to the phosphate buffer solution (pH 6.5) so that the concentration increased from 10 mM to 400 mM (75%) for 15 minutes. The absorbance at 280 nm of the eluent (liquid flowing out from the inside of the adsorption apparatus) was measured. The result of the elution in Example 1 is shown in Fig. 2. The elution positions of both the acidic protein and the basic protein did not change after the preservation.

### (Comparative Example 1)

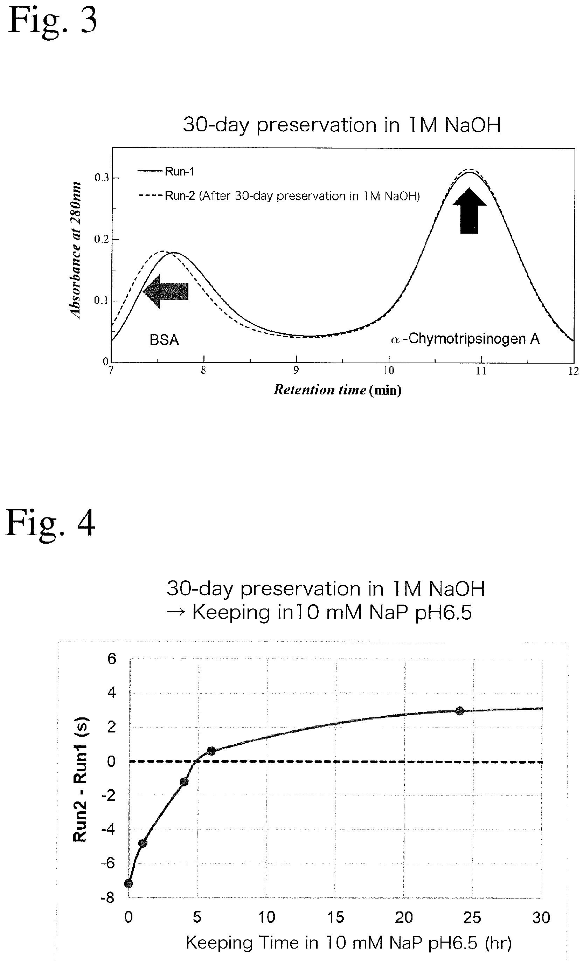

The same experiment as in Example 1 was conducted except that the adsorbents were preserved in 1M NaOH in place of 10 mM HEPES buffer solution (pH 8.0) containing 20% ethanol in the step 4A, and that a step of substituting the preservation liquid in the adsorption apparatus with 0.4 M NaP (pH 6.5) was added between the step 4A and the step 1B. It is noted that in Comparative Example 1, a step of keeping the adsorbents immersed in the phosphate buffer solution, which was carried out in the conventional techniques, was not carried out. The result of the elution in Comparative Example 1 is shown in Fig. 3. It was found that the elution position of the acidic protein had changed after the preservation.

### (Comparative Example 2)

The same experiment as in Example 1 was conducted except that the adsorbents were preserved in 1M NaOH in place of 10 mM HEPES buffer solution (pH 8.0) containing 20% ethanol in the step 4A, and that a step of substituting the preservation liquid in the adsorption apparatus with 10 mM NaP (pH 6.5) and keeping for a certain period of an hour or more was added after the step 4A. The result of the elution in Comparative Example 2 is shown in Fig. 4. Comparative example 2 revealed that the moderating effect was larger as the keeping time was longer.

### (Comparative Example 3)

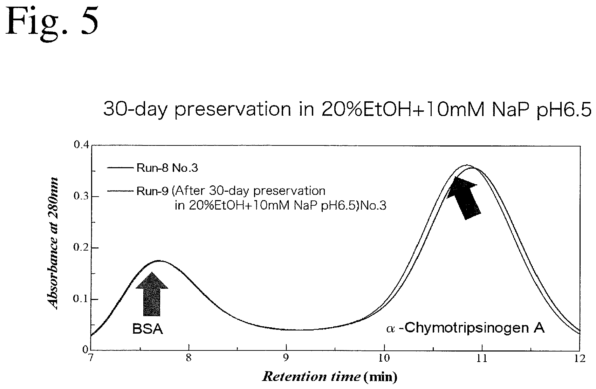

The same experiment as in Example 1 was conducted except that the adsorbents were preserved in 10 mM NaP (pH 6.5) containing 20% ethanol in place of 10 mM HEPES buffer solution (pH 8.0) containing 20% ethanol in the step 4A. The result of the elution in Comparative Example 3 is shown in Fig. 5. In comparison with before the preservation of the column, the elution position of the acidic protein did not change, but the elution position of the basic protein had changed.

Typical embodiments of separation experiments in the above example and comparative examples are intelligibly summarized in the following tables, without intention of restriction thereto. As described in Example 1, a step of washing the adsorbents with water or low-concentration NaP (phosphate buffer solution) may be added before substituting the pH of the adsorbents with "high-concentration NaP" and preserving the adsorbents in "20% EtOH + HEPES" in

### Table 1.

**Table 1**

| 20% EtOH + HEPES Preservation | | |
|---|---|---|
| Step No. | Step | Buffering agent |
| 1 | Column Initial Washing | NaOH |
| 2 | pH Substitution | High-concentration NaP |
| 3 | Equilibration | Low-concentration NaP / Low-concentration Good buffer / Other Low-concentration Buffer |
| 4 | Sample Supply | Low-concentration NaP / Low-concentration Good buffer / Other Low-concentration Buffer |
| 5 | Gradient Starting | A-Buffer: The Same Buffer as in Equilibration Step |
| 6 | | B-Buffer: The Same Buffer as in Equilibration Step + High-concentration Salt |
| 7 | Washing-1 | High-concentration NaP |
| 8 | Washing-2 | NaOH |
| 9 | pH Substitution | High-concentration NaP |
| 10 | Preservation ↓ Next Separation | 20% EtOH + HEPES |
| 2 | Equilibration | Low-concentration NaP / Low-concentration Good buffer / Other Low-concentration Buffer |
| 3 | Sample Supply | Low-concentration NaP / Low-concentration Good buffer / Other Low-concentration Buffer |
| 4 | Gradient Starting | A-Buffer: The Same Buffer as in Equilibration Step |
| 5 | | B-Buffer: The Same Buffer as in Equilibration Step + High-concentration Salt |
| 6 | Washing-1 | High-concentration NaP |
| 7 | Washing-2 | NaOH |
| 8 | pH Substitution | High-concentration NaP |
| 9 | Preservation | 20% EtOH + HEPES |

**Table 2**

| Comparative Example 1 (Prior Art) | | |
|---|---|---|
| NaOH Preservation | | |
| Step No. | Step | Buffering agent |
| 1 | Column Initial Washing | NaOH |
| 2 | pH Substitution | High-concentration NaP |
| 3 | Equilibration | Low-concentration NaP / Low-concentration Good buffer / Other Low-concentration Buffer |
| 4 | Sample Supply | Low-concentration NaP / Low-concentration Good buffer / Other Low-concentration Buffer |
| 5 | Gradient Starting | A-Buffer: The Same Buffer as in Equilibration Step |
| 6 | | B-Buffer: The Same Buffer as in Equilibration Step + High-concentration Salt |
| 7 | Washing-1 | High-concentration NaP |
| 8 | Washing-2 | NaOH |
| 9 | Preservation ↓ | Low-concentration NaOH, etc. |
| | Next Separation | |
| 1 | pH Substitution | High-concentration NaP |
| 2 | Equilibration | Low-concentration NaP / Low-concentration Good buffer / Other Low-concentration Buffer |
| 3 | Sample Supply | Low-concentration NaP / Low-concentration Good buffer / Other Low-concentration Buffer |
| 4 | Gradient Starting | A-Buffer: The Same Buffer as in Equilibration Step |
| 5 | | B-Buffer: The Same Buffer as in Equilibration Step + High-concentration Salt |
| 6 | Washing-1 | High-concentration NaP |
| 7 | Washing-2 | NaOH |
| 8 | Preservation | Low-concentration NaOH, etc. |

**Table 3**

| Comparative Example 2 (Patent Reference 2) | | |
|---|---|---|
| Substitution with 10 mM NaP (pH 6.5) and Keeping for an Hour | | |
| Step No. | Step | Buffering agent |
| 1 | Column Initial Washing | NaOH |
| 2 | pH Substitution | High-concentration NaP |
| 3 | Equilibration | Low-concentration NaP / Low-concentration Good buffer / Other Low-concentration Buffer |
| 4 | Sample Supply | Low-concentration NaP / Low-concentration Good buffer / Other Low-concentration Buffer |
| 5 | Gradient Starting | A-Buffer: The Same Buffer as in Equilibration Step |
| 6 | | B-Buffer: The Same Buffer as in Equilibration Step + High-concentration Salt |
| 7 | Washing-1 | High-concentration NaP |
| 8 | Washing-2 | NaOH |
| 9 | Preservation | Low-concentration NaOH, etc. |
| | ↓ | |
| | Next Separation | |
| 1 | pH Substitution | High-concentration NaP |
| 2 | Substitution | Substitution with 10 mM NaP (pH 6.5) |
| 3 | Keeping | Keeping in 10 mM NaP (pH 6.5) for an Hour |
| 4 | Equilibration | Low-concentration NaP / Low-concentration Good buffer / Other Low-concentration Buffer |
| 5 | Sample Supply | Low-concentration NaP / Low-concentration Good buffer / Other Low-concentration Buffer |
| 6 | Gradient Starting | A-Buffer: The Same Buffer as in Equilibration Step |
| 7 | | B-Buffer: The Same Buffer as in Equilibration Step + High-concentration Salt |
| 8 | Washing-1 | High-concentration NaP |
| 9 | Washing-2 | NaOH |
| 10 | Preservation | Low-concentration NaOH, etc. |

**Table 4**

| Comparative Example 3 (Prior Art) | | |
|---|---|---|
| 20% EtOH + Phosphate Buffer Solution Preservation | | |
| Step No. | Step | Buffering agent |
| 1 | Column Initial Washing | NaOH |
| 2 | pH Substitution | High-concentration NaP |
| 3 | Equilibration | Low-concentration NaP / Low-concentration Good buffer / Other Low-concentration Buffer |
| 4 | Sample Supply | Low-concentration NaP / Low-concentration Good buffer / Other Low-concentration Buffer |
| 5 | Gradient Starting | A-Buffer: The Same Buffer as in Equilibration Step |
| 6 | | B-Buffer: The Same Buffer as in Equilibration Step + High-concentration Salt |
| 7 | Washing-1 | High-concentration NaP |
| 8 | Washing-2 | NaOH |
| 9 | Preservation | Low-concentration NaOH, etc. |
| | ↓ | |
| | Next Separation | |
| 2 | Equilibration | Low-concentration NaP / Low-concentration Good buffer / Other Low-concentration Buffer |
| 3 | Sample Supply | Low-concentration NaP / Low-concentration Good buffer / Other Low-concentration Buffer |
| 4 | Gradient Starting | A-Buffer: The Same Buffer as in Equilibration Step |
| 5 | | B-Buffer: The Same Buffer as in Equilibration Step + High-concentration Salt |
| 6 | Washing-1 | High-concentration NaP |
| 7 | Washing-2 | NaOH |
| 8 | Preservation | Low-concentration NaOH, etc. |

In a case where adsorbents were preserved in 1M NaOH for a preservation period of 7 days, as described in Patent Reference 2, when the preservation liquid was substituted with 10 mM NaP (pH 6.5) and the adsorbents were kept therein for an hour, the elution position of BSA was shifted from Run-1 to Run-2 by -5 (s). When adsorbents are preserved in NaOH for a long period of time, an hour or more of the keeping step in 10 mM NaP (pH 6.5) is required, and thus it seems to be necessary to determine the keeping time in 10 mM NaP (pH 6.5) for every NaOH preservation periods, resulting in complicated preparation. On the contrary, according to the purification method of the present invention, because such a keeping step is not required, it is not necessary to determine the keeping time.

According to the purification method of one embodiment of the present invention, change of the adsorption ability of adsorbents composed of calcium phosphate compound by the preservation step is suppressed.

Therefore, the separation position (elution time) to be desorbed from the adsorbents can be maintained almost constant before and after the preservation in the preservation liquid. Accordingly, when the eluate flowing out from the column for liquid chromatography, in which the adsorbents are filled, are fractionated in predetermined amounts to separate charged materials into each eluate fraction, it can be properly suppressed or prevented to change the separation position of the fraction in which the charged material is to be separated before and after the preservation in the preservation liquid. As a result, because it is not necessary to change the preservation liquid and to restore the fraction to be collected, the separation of the charged materials from the sample liquid can be carried out without requiring time and labor.

Herein, numerical values appended with "about" indicate that the numerical values may vary within a consistent range. The numerical values appended with "about" may, for example, have an error range of plus or minus 10%. The error range is more preferably plus or minus 5%, further preferably plus or minus 1%, most preferably zero (the numerical value itself).

### DESCRIPTION OF REFERENCE NUMERALS

- 1:: Adsorption apparatus
- 2:: Column
- 3:: Adsorbent
- 4:: Filter member
- 5:: Filter member
- 20:: Adsorbent filling space
- 21:: Column body
- 22:: Cap
- 23:: Cap
- 24:: Inlet pipe
- 25:: Outlet pipe

## Claims

1. A method for purifying a compound having a charged portion, the method comprising steps of:
preparing a composition containing a compound having a charged portion;
preparing a buffer solution comprising a buffering agent and an alcohol, the buffer containing a calcium phosphate compound at least partially, having a buffer capacity in a range of pH 6.0 to pH 8.0, and being soluble in a polar solvent and insoluble in a non-polar solvent;
preserving the adsorbent in the buffer solution;
adsorbing the compound on the adsorbent by bringing the composition into contact with the adsorbent preserved in the buffer solution; and
separating the compound from the adsorbent by gradient elution.

2. The purification method according to claim 1, wherein the adsorption step is carried out after an equilibration step using the same kind of a buffering agent as that of the buffering agent used in the adsorption step, without carrying out a substitution step of the buffer solution after the preservation step.

3. The purification method according to claim 1, wherein the charged material has a negatively charged portion.

4. The purification method according to claim 1, wherein the charged material has a positively charged portion.

5. The purification method according to claim 1, wherein the buffering agent is selected from the group consisting of MES, ADA, PIPES, ACES, cholamine chloride, BES, TES, HEPES, acetamido glycine, tricine, glycinamide, and bicine.

6. The purification method according to any one of claims 1 to 5, comprising a step of purifying the compound having a charged portion.
